# EUROPÄISCHE PATENTSCHRIFT

(11) **EP 3 515 372 B1**
(45) Veröffentlichungstag und Bekanntmachung des Hinweises auf die Patenterteilung: **11.11.2020**
(21) Anmeldenummer: 17772669.2
(22) Anmeldetag: 20.09.2017
(51) Int. Cl.: A61F 2/78, A61F 2/50

(54) **PROTHESENKUPPLUNG FÜR EINE PROTHESE SOWIE PROTHESE MIT DER PROTHESENKUPPLUNG**
PROSTHESIS COUPLING FOR A PROSTHESIS, AND PROSTHESIS WITH THE PROSTHESIS COUPLING
DISPOSITIF D'ACCOUPLEMENT DE PROTHÈSE CONÇU POUR UNE PROTHÈSE, ET PROTHÈSE ÉQUIPÉE DE CE DISPOSITIF D'ACCOUPLEMENT DE PROTHÈSE

(30) Priorität: 26.09.2016 CH 12542016
(43) Veröffentlichungstag der Anmeldung: 31.07.2019
(73) Patentinhaber: Appsocial.org Stiftung, 8032 Zürich (CH)
(72) Erfinder: SCHOLLENBERGER, Fabian, 8127 Forch (CH)
(74) Vertreter: Keller Schneider Patent- und Markenanwälte AG (Zürich)
(86) Internationale Anmeldenummer: PCT/EP2017/073741
(87) Internationale Veröffentlichungsnummer: WO 2018/054954

(56) Entgegenhaltungen:
- GB-A- 2 278 281
- GB-A- 2 479 532
- US-A1- 2011 015 761

## Beschreibung

### Technisches Gebiet

Die vorliegende Erfindung beschreibt eine Prothesenkupplung für eine Prothese, sowie eine Prothese mit der Prothesenkupplung gemäss Oberbegriff des ersten Patentanspruches und eine Prothese mit einer derartigen Prothesenkupplung.

### Stand der Technik

Bekannt ist beispielsweise aus Dokument US 2011/0015761 A1 eine Prothesenkupplung beziehungsweise ein prothetischer Adapter, welcher an einem Extremitätenstumpf sowie an einem Prothesenmodul, hier einem Prothesenfuss, anbringbar ist. Die Prothesenkupplung umfasst eine erste Komponente zum Anbringen der Prothesenkupplung an einem zur Aufnahme eines Extremitätenstumpfs ausgebildeten Prothesenschaft, und eine zweite Komponente zum Anbringen der Prothesenkupplung am Prothesenmodul. Sowohl an der ersten wie auch an der zweiten Komponente wird technisch umständlich mittels einer Vielzahl von Schrauben eine axiale Beweglichkeit sowie eine mögliche Verdrehung des Prothesenschafts relativ zum Prothesenmodul verhindert.

Ebenfalls ist die sogenannte "Michelangelo Hand" der Firma Otto Bock Healthcare bekannt. Auf einer Seite ist eine Prothesenkupplung mit dem Prothesenschaft verschraubbar. Im Weiteren ist die Prothesenkupplung durch Drücken eines Knopfs am Prothesenschaft betätigbar und dadurch auf der anderen Seite dieser Prothesenkupplung das Prothesenmodul, hier in Form einer Handprothese, vom Prothesenschaft bzw. von dem am Prothesenschaft fixierten Prothesenkupplungabschnitt lösbar, während durch einen Einrastmechanismus beide Prothesenkupplungsabschnitte und damit der Prothesenschaft mit dem Prothesenmodul verbindbar. Die "Michelangelo Hand" ist äusserst robust ausgebildet und zwangsläufig schwer. Da diese Prothese verschiedenste Bewegungen ausführen können soll und auch fremdbetätigbar ist, ist diese massive Ausgestaltung notwendig. Zum einen ist der Prothesenschaft massiv ausgestaltet und zum anderen ist das Prothesenmodul komplex. Auch die Prothesenkupplungsabschnitte sind entsprechend robust und technisch aufwändig gestaltet, was dazu führt, dass nicht jeder Prothesenträger in der Lage ist das Prothesenmodul an- und abzukoppeln. Die bekannten Prothesenkupplungsabschnitte sind derart schwer, dass der Tragekomfort verringert ist.

Eine Prothesenkupplung gemäß des Oberbegriffs von Anspruch 1 ist aus der Patentschrift GB 2 278 281 A bekannt. Es ist ein anhaltendes Bedürfnis, die Handhabung für den Prothesenträger noch weiter zu verbessern und ein vereinfachtes An- und Abkoppeln zu gewährleisten. Vor allem, wenn ein Prothesenträger mehrere Prothesenmodule besitzt und diese öfters wechseln möchte.

### Darstellung der Erfindung

Die vorliegende Erfindung hat sich deshalb zur Aufgabe gestellt, eine Prothesenkupplung für eine Prothese, sowie eine Prothese mit der Prothesenkupplung bereitzustellen, welche eine vereinfachte Handhabung durch einen Prothesenträger erlaubt. Ein Prothesenmodul soll derart mittels Prothesenkupplung an einem Prothesenschaft befestigt werden, dass ein Herabfallen des Prothesenmoduls während des An- und Abkuppelns mit einer Hand verhindert wird.

Diese Aufgabe wird durch eine Prothesenkupplung für eine Prothese mit den Merkmalen des Patentanspruches 1 gelöst.

Erfindungsgemäss umfasst die Prothesenkupplung einen ersten Kupplungsabschnitt mit zwei in einem Verschlusssockel beweglich gehaltenen Verschlusselementen sowie einen zweiten Kupplungsabschnitt mit einem Verbindungsbauteil, wobei das Verbindungsbauteil in einem korrespondierenden Aufnahmebereich zwischen den zwei Verschlusselementen aufnehmbar und in Position haltbar ist, und wobei die Verschlusselemente mittels eines, von einer Offenstellung in eine Schliessstellung beweglichen, Arretierrings in der Beweglichkeit derart in einer Schliessstellung blockierbar sind, so dass mittels der Prothesenkupplung zwischen dem Prothesenmodul und dem Prothesenschaft eine lösbare Verbindung herstellbar ist.

Es hat sich eine verbesserte Handhabung für den Benutzer gezeigt. Insbesondere konnte eine einhändig und dadurch durch den Benutzer selbst und in einfacher Weise bedienbare prothetische Schnellverschlusskupplung erzielt werden.

Das Prothesenmodul wird nach Aufnahme des daran fixierten Verbindungsbauteils im Aufnahmebereich zwischen den Verschlusselementen bereits provisorisch in Position gehalten, bevor mittels des Arretierrings eine gesicherte Verbindung zwischen Prothesenmodul und Prothesenschaft hergestellt wird. Dadurch ist die Prothesenkupplung risikolos mit einer Hand bedienbar.

Insbesondere kann mittels Prothesenkupplung in einfacher Weise je nach Bedarf das gerade verwendete Prothesenmodul durch ein auf eine jeweilige andere Anwendung angepasstes Prothesenmodul durch den Benutzer selbst ohne die Hilfe einer weiteren Person ausgetauscht werden, wobei dieser Austausch auch mühelos von Kindern durchführbar ist.

Das Verbindungsbauteil kann in Form eines Sechskantelements oder in einer beliebigen anderen Vielecksform gewählt werden, wodurch die Anzahl möglicher radialer Stellungen des Prothesenmoduls beliebig variiert werden kann.

Vorzugsweise umfasst das Verbindungsbauteil nebst dem Sechskantelement ein, vorzugsweise im Wesentlichen konzentrisch zum Sechskantelement ausgerichtetes, Scheibenelement, wobei das Sechskantelement an das Scheibenelement angeformt ist und zwischen dem Sechskantelement und dem Scheibenelement eine Sollbruchstelle ausgebildet ist. Bei den eingangs erwähnten, bekannten Prothesenkupplungen für Handprothesen beziehungsweise Fussprothesen wird im Falle eines harten Schlages auf das Prothesenmodul dieses von der Prothesenkupplung weitergegeben und kann dabei nebst dem Prothesenmodul beziehungsweise der Prothesenkupplung sogar der Prothesenschaft beschädigt und / oder der Extremitätenstumpf des Benutzers verletzt werden. Durch die Bereitstellung einer Sollbruchstelle an der erfindungsgemässen Prothesenkupplung wird vorteilhaft bei einem harten Schlag das Prothesenmodul an einer gewünschten Stelle entkoppelt, wodurch der Extremitätenstumpf des Benutzers vor Verletzung beziehungsweise der Prothesenschaft oder das Prothesenmodul vor Beschädigungen geschützt werden. Insbesondere handelt es sich bei diesem Verbindungsbauteil um ein einfaches und kostengünstiges Bauteil, welches vom Prothesenträger selber ohne besondere Fachkenntnis ersetzt werden kann, wodurch Reparaturkosten beziehungsweise Reparaturzeiten entfallen.

Im Sinne der vorliegenden Erfindung wird unter einer Sollbruchstelle insbesondere ein durch konstruktive oder mechanische beziehungsweise physikalische Massnahmen oder Auslegungen vorgesehenes Konstruktionselement, hier das Verbindungsbauteil, verstanden, wobei im Schadensfall oder Überlastfall dieses Konstruktionselement gezielt und vorhersagbar versagt, um hierdurch den möglichen Schaden im Gesamtsystem klein zu halten. Ein Überlastfall kann beispielsweise mittels Zug, Torsion und / oder Biegung auftreten.

Besonders bevorzugt umfasst die Prothesenkupplung ein zwischen dem Prothesenmodul und dem Scheibenelement anbringbares Rasterscheibenelement, wobei das Rasterscheibenelement zu einer korrespondierenden, dem Rasterscheibenelement zugewandten Rasterung des Scheibenelements hin montierbar ist, so dass zusätzlich die radiale Verstellbarkeit des am Scheibenelement angeformten Sechskantelements gegenüber dem Prothesenmodul ermöglicht wird.

### Kurze Beschreibung der Zeichnungen

Ein bevorzugtes Ausführungsbeispiel des Erfindungsgegenstandes wird nachstehend im Zusammenhang mit den anliegenden Zeichnungen beschrieben. Es zeigen:
- Fig. 1: eine erste bevorzugte Ausführungsform der erfindungsgemässen, in einem Prothesenschaft aufgenommenen Prothesenkupplung für eine Handprothese in einem Längsschnitt;
- Fig. 2: eine perspektivische Ansicht der ersten bevorzugten Ausführungsform der erfindungsgemässen Prothesenkupplung mit einem an der Prothese fixierten Sechskantelement;
- Fig. 3: eine perspektivische Ansicht der Prothesenkupplung im in einer Handprothese und dem Prothesenschaft aufgenommenen Zustand;
- Fig. 4: eine Explosionszeichnung der ersten bevorzugten Ausführungsform der erfindungsgemässen Prothesenkupplung;
- Fig. 5a: eine perspektivische Ansicht des ersten Kupplungsabschnitts im zusammengesetzten Zustand mit dem Arretierring in einer Offenstellung vor dem Einführen des zweiten Kupplungsabschnittes;
- Fig. 5b: eine perspektivische Ansicht der Prothesenkupplung gemäss Figur 5a mit dem Arretierring in einer Schliessstellung ohne zweiten Kupplungsabschnitt;
- Fig. 6: eine Explosionszeichnung der zweiten bevorzugten Ausführungsform der erfindungsgemässen Prothesenkupplung für eine Handprothese;
- Fig. 7a: eine perspektivische Ansicht eines Verbindungsbauteils der zweiten bevorzugten Ausführungsform erfindungsgemässen Prothesenkupplung mit einer zwischen Sechskantelement und Scheibenelement ausgebildeten Sollbruchstelle;
- Fig. 7b-d: jeweils in einer Schnittansicht und einer Aufsicht ein Verbindungsbauteil der zweiten bevorzugten Ausführungsform mit unterschiedlich dimensionierten Sollbruchstellen zwischen Sechskantelement und Scheibenelement.

### Beschreibung

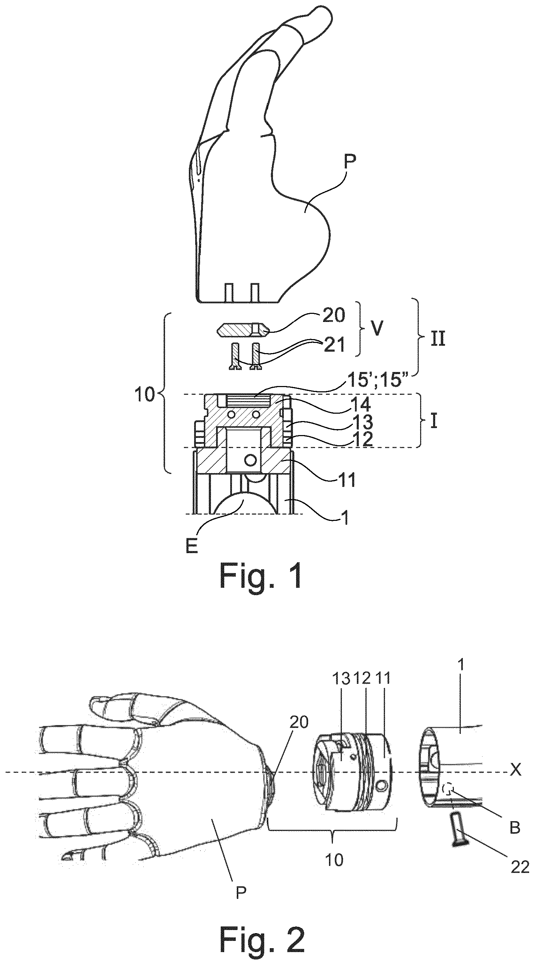

Fig. 1 zeigt eine erste bevorzugte Ausführungsform einer Prothesenkupplung 10, umfassend einen ersten Kupplungsabschnitt I und einen zweiten Kupplungsabschnitt II, welche miteinander lösbar verbindbar an- und abkuppelbar sind. Der erste Kupplungsabschnitt I ist hier an einem Prothesenschaft 1 befestigt, wobei der Prothesenschaft 1 an einem Extremitätenstumpf E des Benutzers beziehungsweise Prothesenträgers fixiert ist. Als Extremitätenstumpf E kann ein Arm- oder Beinstumpf und als Prothesenmodul P kann eine Hand- oder Fussprothese verstanden werden. Der zweite Kupplungsabschnitt II ist an einem Prothesenmodul P befestigt. Durch ein Zusammenwirken des ersten und zweiten Kupplungsabschnittes I, II werden Prothesenmodul P und Prothesenschaft 1 miteinander lösbar verbunden.

Die Prothesenkupplung 10 bzw. der erste und zweite Kupplungsabschnitt I, II umfassen einen Befestigungsring 11, ein Druckfederelement 12, einen Arretierring 13, einen Verschlusssockel 14 sowie ein mit dem Verschlusssockel 14 kompatibles Verbindungsbauteil V.

Hier umfasst der erste Kupplungsabschnitt I den Befestigungsring 11, das Druckfederelement 12, den Arretierring 13 und den Verschlusssockel 14. Im Verschlusssockel 14 sind beweglich gehaltene Verschlusselemente 15', 15" angeordnet, welche ebenfalls Teil des ersten Kupplungsabschnittes I sind. Der Befestigungsring 11 der Prothesenkupplung 10 ist hier in einem Aufnahmebereich des Prothesenschafts 1 aufgenommen und darin durch geeignete Mittel befestigt beziehungsweise fixiert. Dies kann beispielsweise über eine Klebeverbindung, Schraubverbindung, mittels Formschluss und / oder eine weitere Verbindungsart erfolgen. Durch den Befestigungsring 11 wird der Verschlusssockel 14 und am Verschlusssockel 14 bewegbar gelagertes Druckfederelement 12 und der bewegbar gelagerter Arretierring 13 gehalten.

Auf der dem Prothesenmodul P zugewandten Seite ist hier beispielhaft der Befestigungsring 11 abschnittsweise in dem Verschlusssockel 14 aufnehmbar und mit geeigneten Mitteln darin fixierbar. Neben einer Klebeverbindung oder Schraubverbindung kann eine formschlüssige Verbindung gewählt sein. Das Druckfederelement 12 sowie der Arretierring 13 umschliessen den Verschlusssockel 14 direkt.

Der zweite Kupplungsabschnitt II umfasst das Verbindungsbauteil V, welches hier als Mehrkant- bzw. Sechskantelement 20 ausgebildet ist. Die Querschnittsfläche des Verbindungsbauteils V ist als Polygon mit mindestens drei Eckpunkten ausführbar. Das Sechskantelement 20 umfasst Bohrungen, durch welche Fixierschrauben 21 führbar sind und eine Befestigung am Prothesenmodul P, hier in Form einer Handprothese, möglich ist. Die Bohrungen sind hier konzentrisch das Mehrkant- bzw. Sechskantelement 20 querend angeordnet.

Durch Ankupplung des ersten Kupplungsabschnittes I mit dem zweiten Kupplungsabschnitt II kann hier die Handprothese P lösbar mit dem Prothesenschaft 1 verbunden werden. Dabei kann die Prothesenkupplung 10 wie in Figur 1 gezeigt, derart orientiert sein, dass der erste Kupplungsabschnitt I am Prothesenschaft 1 befestigt ist und der zweite Kupplungsabschnitt II an der Handprothese P befestigt ist. Die Prothesenkupplung 10 kann aber auch um 180° rotiert zwischen Handprothese P und Prothesenschaft 1 angeordnet sein.

Von hier an und im Folgenden bezeichnen gleiche Referenzzeichen gleiche Komponenten in den Figuren.

Fig. 2 zeigt die erste bevorzugte Ausführungsform der Prothesenkupplung 10 mit einem an dem Prothesenmodul P fixierten Sechskantelement 20. Wie in Fig. 2 angedeutet wird üblicherweise nach Aufnahme des Befestigungsrings 11 der Prothesenkupplung 10 im Aufnahmebereich des Prothesenschafts 1 eine Fixierung mittels Fixierschrauben erzielt, indem die Fixierschrauben 22 die Wand des Prothesenschafts 1 durch eine Bohrung B queren und im Befestigungsring 11 der Prothesenkupplung 10 verankert werden können. Hier ist im Befestigungsring 11 ebenfalls eine Aussparung zur Befestigung vorgesehen.

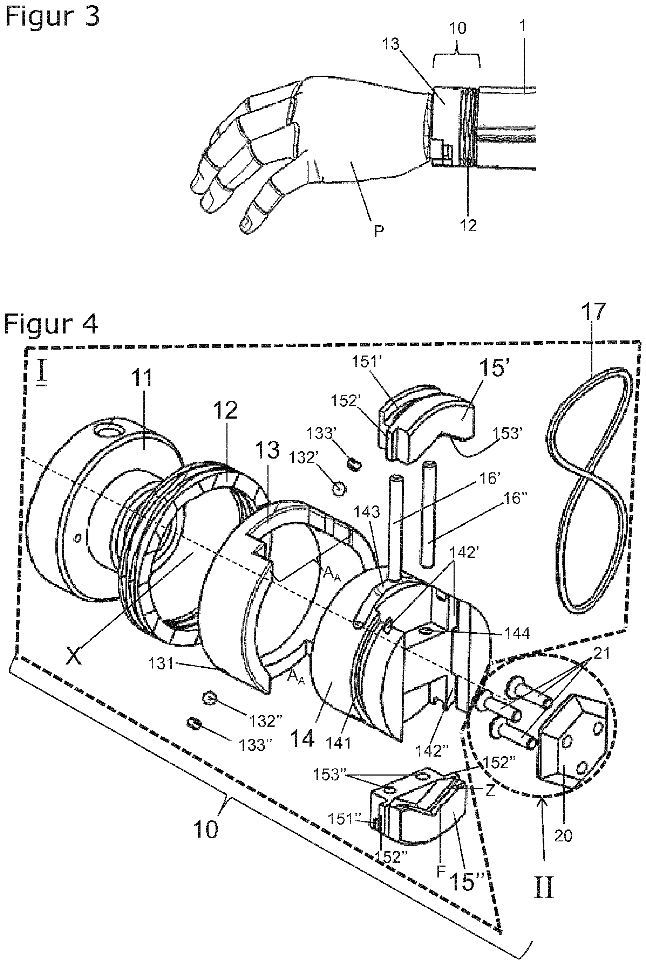

Fig. 3 zeigt die erste bevorzugte Ausführungsform der Prothesenkupplung 10 nach Ankupplung des Prothesenmoduls P, insbesondere in Form einer Handprothese, am Prothesenschaft 1, wobei die Abschnitte I, II der Prothesenkupplung 10 miteinander gekuppelt sind. Das Verbindungsbauteil V ist dabei durch die Bauteile des zweiten Kupplungsabschnittes I lösbar gehalten, wobei eine Arretierung erfolgt ist.

Fig. 4 zeigt eine Explosionszeichnung der ersten bevorzugten Ausführungsform der Prothesenkupplung 10. Mittels Befestigungsring 11 wird der erste Kupplungsabschnitt I beispielsweise am Prothesenschaft 1 ortsfest und rotationsfest gehalten befestigt. Am Befestigungsring 11 wird der Verschlusssockel 14 ebenfalls orts- und rotationsfest angeordnet.

Zwischen dem Verschlusssockel 14 werden das ringförmig ausgeführte Druckfederelement 12 und der Arretierring 13 angeordnet, wobei Druckfederelement 12 und Arretierring 13 bewegbar gelagert werden. Das Druckfederelement 12 lagert zwischen einer Fläche des Befestigungsringes 11 und des Arretierringes 13 und kann auf den Arretierring 13 einwirken. Während das Druckfederelement 12 in axialer Richtung parallel zur Längsachse X auslenkbar ist, ist der Arretierring 13 drehbar und linear bewegbar gelagert. Das Druckfederelement 12 drückt im montierten Zustand den Arretierring 13 vom Befestigungsring 11 axial weg parallel zur Längsachse X.

Der Arretierring 13 umfasst Führungskugelaufnahmen 131, in der Führungskugelaufnahme 131 aufnehmbare Führungskugeln 132', 132" sowie Gewindestifte 133',133" zur Fixierung der aufgenommenen Führungskugeln 132',132". Entlang seines Kreisumfanges weist der Arretierring 13 zwei gegenüberliegende Aussparungen A_{A} innerhalb der Arretierringwand auf.

Der Verschlusssockel 14 ist in den Innendurchmesser des Arretierrings 13 einführbar, sodass der Arretierring 13 den Verschlusssockel 14 umschliesst. Auf der äusseren Umfangsfläche des Verschlusssockels 14 ist eine Halterille 141 und mindestens ein bogenförmiger Führungskanal 143 ausgespart. Der Führungskanal 143 verläuft entlang einer Kurve auf der zylindrischen Umfangsfläche des Verschlusssockels 14 zwischen zwei Anschlägen, welche den Anfang und das Ende des Führungskanals 143 bilden.

Aus dem Körper des Verschlusssockels 14 sind mehrere Führungsnuten 142', 142" und den Verschlusssockel 14 querende Führungsstiftaufnahmen 144 ausgespart.

Ist der Arretierring 13 mit dem Verschlusssockel 14 wirkverbunden, dann kann die mindestens eine Führungskugel 132', 132" im Führungskanal 143 des Verschlusssockels 14 rollen, wenn der Arretierring 13 relativ zum Verschlusssockel 14 rotiert wird. Der Einfachheit halber ist in Figur 4 nur ein Führungskanal 143 gezeigt, in welchem eine erste Führungskugel 132' rollend lagerbar ist. Die Führungskugeln 132', 132" sind in, mit einem Innengewinde versehenen, Führungskugelaufnahmen 131 des Arretierrings 13 aufnehmbar und dort mit Gewindestiften 133', 133" am Arretierring 13 bzw. zwischen Arretierring 13 und äusserer Umfangsfläche des Verschlusssockels 14 rollbar fixiert. Durch den Verlauf des Führungskanals 143 wird hier eine lineare und rotative Bewegung des Arretierrings 13 erreicht.

Im Weiteren ist hier an den bewegbar lagerbaren Verschlusselementen 15', 15" jeweils ein seitlich wegragender Führungssteg 152', 152" angeordnet. Der jeweils wegragende Führungssteg 152', 152" ist in der jeweils korrespondierenden Führungsnut 142', 142" des Verschlusssockels 14 aufnehmbar und darin beweglich. Durch das Eingreifen der Führungsstege 152', 152" der Verschlusselemente 15', 15" in die auf gegenüberliegenden Seiten am Verschlusssockel 14 eingeformten Führungsnuten 142', 142" ist eine geführte Einführung der Verschlusselemente 15', 15" in jeweils eine Verschlusssockelaussparung für jedes Verschlusselement 15', 15" möglich.

In den zwei ersten Führungsstiftaufnahmen 144 im Verschlusssockel 14 sind zwei Führungsstifte 16', 16" aufnehmbar. Die Führungsstifte 16', 16" queren den Verschlusssockel 14 durch die ersten Führungsstiftaufnahmen 144 und sind zumindest abschnittsweise in zweiten und dritten Führungsstiftaufnahmen 153', 153" in den Verschlusselementen 15', 15" aufnehmbar. Die Verschlusselemente 15', 15" sind auf den Führungsstiften 16', 16" linear bewegbar gelagert, wobei die Führungsstifte 16', 16" unterschiedlich tief in die zweiten und dritten Führungsstiftaufnahmen 153', 153" eintauchen können. Die Länge der Führungsstifte 16', 16" und die Grösse der Verschlusssockelaussparungen sowie der Verschlusselemente 15', 15" selbst müssen derart bemessen sein, dass der äussere Umfang der Verschlusselemente 15', 15" mindestens mit dem äusseren Umfang des Verschlusssockels 14 abschliessen kann.

Für eine provisorische Sicherung des zweiten Kupplungsabschnittes II im ersten Kupplungsabschnitt I wird ein Rückstellmittel 17, hier in Form eines Gummibands 17 genutzt. Das Gummiband 17 umgibt den Verschlusssockel 14 und darin gehaltene Verschlusselemente 15', 15". Hier umschliesst das Gummiband 17 Verschlusssockel 14 und Verschlusselemente 15', 15", indem es in der ersten Halterrille 141 im Verschlusssockel 14, sowie in einer zweite Halterille 151' des Verschlusselements 15' und einer dritten Halterille 151" des Verschlusselements 15" zu liegen kommt. Im zusammengesetzten Zustand begrenzt das Gummiband 17 die Bewegungsmöglichkeiten der Verschlusselemente 15', 15" auf den Führungsstiften 16', 16" in einer Richtung senkrecht zur Längsachse X.

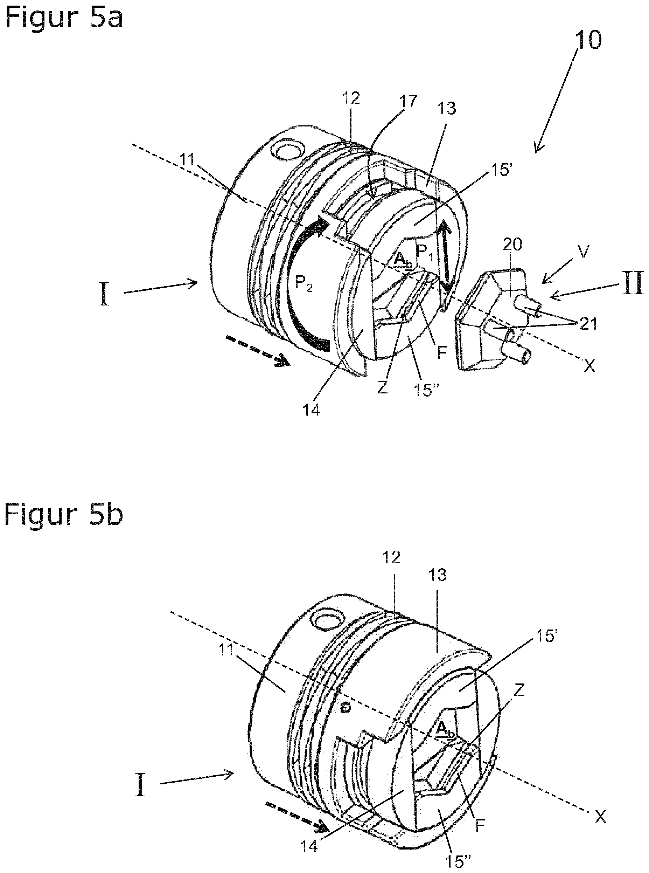

Fig. 5a stellt die erste bevorzugte Ausführungsform der Prothesenkupplung 10 dar, wobei der erste Kupplungsabschnitt I im zusammengesetzten Zustand mit Arretierring 13 in einer Offenstellung gezeigt ist und die Verschlusselementen 15',15" vom Gummiband 17 gehalten in einer Ausgangsstellung angeordnet sind. In der Ausgangsstellung sind die Verschlusselementen 15',15" in ihren Verschlusssockelaussparungen gehalten.

Auf gegenüberliegenden Seiten des Arretierrings 13 sind die Aussparungen A_{A} derart ausgebildet, dass in der Ausgangsstellung die Verschlusselemente 15',15" in einer Richtung senkrecht zur Längsachse X durch die Aussparungen A_{A} mindestens teilweise querend bewegbar sind.

Dabei bilden die Verschlusselemente 15',15" und Innenwände des Verschlusssockels 14 einen Aufnahmebereich A_{b}. Für das hier als Sechskantelement 20 ausgestaltete Verbindungsbauteil V zeigt der Aufnahmebereich A_{b} zwischen den Verschlusselementen 15', 15" und Innenwänden des Verschlusssockels 14 ebenfalls einen sechseckigen und damit einen zum Sechskantelement 20 korrespondierenden Querschnitt.

Wird das Sechskantelement 20 in den Aufnahmebereich A_{b} zwischen den Verschlusselementen 15',15" der Prothesenkupplung 10 in Richtung Längsachse X gedrückt, bewegen sich die Verschlusselemente 15', 15" von der in Fig. 5a gezeigten Ausgangsstellung entlang der jeweiligen Führungsnut in Pfeilrichtungen P₁ auseinander. Gegen das vorgespannte Gummiband 17 können die Verschlusselemente 15', 15" jeweils linear vom Zentrum bzw. der Längsachse X wegbewegt werden. Sobald das Sechskantelement 20 vollständig im Aufnahmebereich A_{b} der Prothesenkupplung 10 aufgenommen ist, werden die Verschlusselemente 15', 15" aufgrund der Elastizität des Gummibandes 17 wieder in die Ausgangsstellung und damit zurück in Richtung der Längsachse X zurückgelenkt, womit eine provisorische Halterung des Verbindungsbauteils V im ersten Kupplungsabschnitt I erreicht ist. Die Öffnungs- und Schliessbewegung der Verschlusselemente 15', 15" erfolgen in einer Ebene senkrecht zur Längsachse X entlang des Doppelpfeils P₁. Durch die Formgebung des Verbindungsbauteils V und den Aufnahmebereich A_{b} ist eine verdrehgesicherte Lagerung des zweiten Kupplungsabschnittes II im ersten Kupplungsabschnitt I erreichbar.

Wie in Fig. 4 sowie in den Fig. 5a und 5b ersichtlich weist vorzugsweise das Sechskantelement 20 im Randbereich beidseitig abgeschrägte Kanten auf. Hierzu korrespondierend weisen vorzugsweise die Verschlusselemente 15',15" jeweils eine Fase F auf, um die Aufnahme des Sechskantelements 20 in den Aufnahmebereich A_{b} zu erleichtern bzw. einen Hinterschnitt zu erzeugen, sodass das Sechskantelement 20 nicht herausrutschen kann. Die Fase F der Verschlusselemente 15',15" umgreift die abgeschrägten Kanten des Sechskantelementes 20, wenn das Verbindungsbauteil V in den Aufnahmebereich A_{b} eingesteckt ist.

Die Verschlusselemente 15', 15" weisen hier optional eine zu den abgeschrägten Kanten des Sechskantelements 20 korrespondierende Zentriernut Z auf, wodurch ein im Aufnahmebereich A_{b} vollständig aufgenommenes Sechskantelement 20 nahezu unbewegbar und verdrehgesichert gehalten wird.

Wenn das Verbindungsbauteil V in den Aufnahmebereich A_{b} eingeführt ist und von den Verschlusselemente 15', 15" gegen die Spannung des Gummibandes 17 ortsfest gehalten wird, ist eine provisorische Halterung erreicht. Das Verbindungsbauteil V und das beispielsweise daran befestigte Prothesenmodul P sind mit dem ersten Kupplungsabschnitt I dann provisorisch verbunden. Die Elastizität und Stärke des Gummibandes 17 ist entsprechend zu wählen, dass diese provisorische Halterung erreichbar ist. Die Verschlusselemente 15', 15" haben die Funktion von Klemmbacken, die das Verbindungsbauteil V im Aufnahmebereich A_{b} halten, während der Arretierring 13 noch in der Offenstellung ist. Der Prothesenträger kann aufgrund der provisorischen Halterung das am Verbindungsbauteil V befestigte Prothesenmodul P loslassen und muss kein Herabfallen befürchten. Entsprechend hat der Prothesenträger die intakte Hand frei und kann unbesorgt weitere Aktionen durchführen.

Nachdem der zweite Kupplungsabschnitt II in den Aufnahmebereich A_{b} eingebracht wurde und darin provisorisch gehalten wird, können erster und zweiter Kupplungsabschnitt I, II fix miteinander verbunden werden. Dazu ist der Arretierring 13 in eine Schliessstellung bringbar.

Durch Drehung des Arretierrings 13, hier beispielsweise um 90 Grad in Pfeilrichtung P₂ um die Längsachse X, wird der Arretierring 13 von der Offenstellung in die in Fig. 5b gezeigte Schliessstellung des Arretierrings 13 gebracht. Der Übersichtlichkeit halber ist das Verbindungsbauteil V in Figur 5b weggelassen, damit der Verschlusssockel 14 und der zentrisch im Verschlusssockel 14 angeordnete Aufnahmebereich A_{b} nicht verdeckt sind.

Der Arretierring 13 wird durch die mindestens eine Führungskugel 132 im mindestens einen Führungskanal 143 geführt verdreht, wobei eine lineare axiale Verschiebung gegen das Druckfederelement 12 und eine Rotationsbewegung um die Längsachse X resultiert. Diese Bewegung erfolgt aufgrund des bogenförmigen Verlaufs des Führungskanals 143 in welchem die Führungskugel 132 rollt. Die vom Druckfederelement 12 erzeugte Gegenkraft wird während der Drehung des Arretierringes 13 überwunden, während die mindestens eine Führungskugel 132', 132" entlang des bogenförmigen Führungskanals 143 rollt. Diese Drehbewegung erfolgt bis die mindestens eine Führungskugel 132', 132" von einem Ausgangspunkt bis zu einem Anschlagspunkt gerollt ist. Nach vollzogener Drehung, hier beispielsweise um 90 Grad, bis zu einem Anschlagspunkt am Ende des Führungskanals 143 wird darauf der Arretierring 13 in der in Fig. 5b gezeigten Schliessstellung gehalten. Dabei wirkt eine Gegenkraft des Druckfederelements 12 auf den Arretierring 13, was durch den gestrichelten Pfeil angedeutet ist. Ein vorher provisorisch gehaltenes Verbindungsbauteil V ist nun im Aufnahmebereich A_{b} arretiert gehalten. In dieser Schliessstellung sind die Verschlusselemente 15',15" radial, insbesondere in der Beweglichkeit in Pfeilrichtungen P₁, blockiert, sodass das im Aufnahmebereich A_{b} zwischen den Verschlusselementen 15', 15" befindliche Sechskantelement 20 durch den Arretierring 13 in der Schliessstellung durch Form- und / oder Kraftschluss fixiert ist.

Durch die Wahl eines Sechskantelements 20 kann vorteilhaft das als Verbindungselement V und das daran befestigte Prothesenmodul P in sechs möglichen radialen Stellungen mittels erstem Kupplungsabschnitt I an den Prothesenschaft 1 gekoppelt werden.

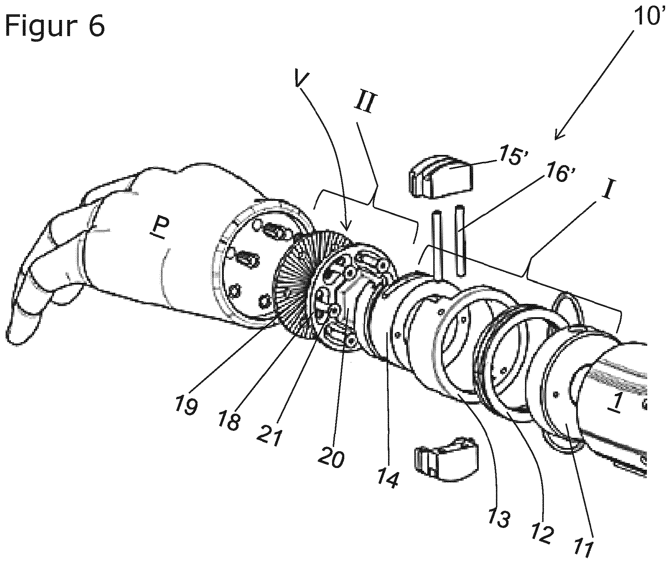

Fig. 6 zeigt eine zweite bevorzugte Ausführungsform der Prothesenkupplung 10' für ein Prothesenmodul P, hier in Form einer Handprothese, wobei identische Bauteile mit gleichen Bezugszeichen gekennzeichnet sind. Auch hier ist eine provisorische Halterung des Verbindungsbauteils V im ersten Kupplungsabschnitt I und eine anschliessende Arretierung des Arretierringes 13 in einer Schliessstellung möglich.
Im Unterschied zu der in den Fig. 1 bis 5b gezeigten, ersten bevorzugten Ausführungsform weist bei dieser Ausführungsform das Verbindungsbauteil V nebst dem Sechskantelement 20 ein Scheibenelement 18 auf, wobei das Sechskantelement 20 an das Scheibenelement 18 angeformt ist und zwischen dem Sechskantelement 20 und dem Scheibenelement 18 eine Sollbruchstelle ausgebildet ist. Wie in Fig. 6 ersichtlich sind bei dieser zweiten bevorzugten Ausführungsform die Fixierschrauben 21 nur durch Bohrungen im Scheibenelement 18 geführt, während das Sechskantelement 20 selbst keine Bohrungen umfasst. Nebst diesem Unterschied entspricht hier der strukturelle Aufbau der zweiten Ausführungsform dem strukturellen Aufbau der ersten Ausführungsform der erfindungsgemässen Prothesenkupplung 10. Wenn die Prothesenkupplung 10 das Prothesenmodul P am Prothesenschaft 1 angekuppelt hält, werden bei zu hoher Krafteinwirkung auf das Prothesenmodul P oder den Prothesenschaft 1 die beiden Kupplungsabschnitte I, II voneinander getrennt. Wenn die Krafteinwirkung einen gewissen Wert übersteigt, bricht das Scheibenelement 18 vom Sechskantelement 20 ab. Das Sechskantelement 20 verbleibt dann im Aufnahmebereich A_{b} im Verschlusssockel 14, gehalten von den Verschlusselementen 15', 15". Das Scheibenelement 18 verbleibt fixiert am Prothesenmodul P oder dem Prothesenschaft 1. Der Prothesenträger kann die Prothese wieder instand setzen, in dem ein neues Verbindungsbauteil V mit Scheibenelement 18 und Sechskantelement 20 als erster Kupplungsabschnitt I verwendet wird und das abgebrochene Sechskantelement 20 aus dem Aufnahmebereich A_{b} im Verschlusssockel 14 entfernt wird.

Optional kann wie in Fig. 6 ersichtlich die Prothesenkupplung 10 ein zwischen dem Prothesenmodul P und dem Scheibenelement 18 anbringbares Rasterscheibenelement 19 umfassen. Dabei ist vorzugsweise das Rasterscheibenelement 19 zu einer korrespondierenden, dem Rasterscheibenelement 19 zugewandten Rasterung des Scheibenelements 18 hin montierbar, so dass zusätzlich die radiale Verstellbarkeit des am Scheibenelement 18 angeformten Sechskantelements 20 gegenüber dem Prothesenmodul P ermöglicht wird. Spezifische Prothesenmodule können dank dieser zusätzlichen feinen Positionierung besonders gezielt auf Kundenbedürfnisse angepasst werden. Natürlich könnten auch hier der erste Kupplungsabschnitt I am Prothesensschaft 1 und der zweite Kupplungsabschnitt II am Prothesenmodul P angeordnet sein.

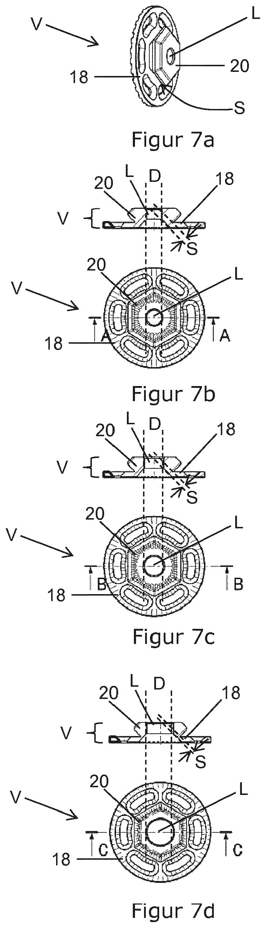

Leicht abgewandelte Mehrkantelemente 20 bzw. Sechskantelemente 20 als Verbindungsbauteil V sind in den Figuren 7 gezeigt. Die Sechskantelemente 20 weisen ein optionales zentrisches Loch L auf, während Befestigungsaussparungen konzentrisch im Scheibenelement 18 angeordnet sind.

Fig. 7a zeigt eine perspektivische Ansicht eines Verbindungsbauteils V für die zweite bevorzugte Ausführungsform der erfindungsgemässen prothetischen Prothesenkupplung 10 mit einer zwischen Sechskantelement 20 und Scheibenelement 18 ausgebildeten Sollbruchstelle S. Die Stärke der Sollbruchstelle kann unterschiedlich, bevorzugt durch Wahl der Materialdicke, der Materialart oder der Gestaltung der Kerbform variiert werden.

Anhand eines in Fig.7b gezeigten Schnitts A-A durch das Verbindungsbauteil V wird die Sollbruchstelle S im Übergangsbereich zwischen Scheibenelement 18 und Sechskantelement 20 erkennbar. Die Wandstärken bzw. der Übergangsbereich vom Sechskantelement 20 zum Scheibenelement 18 ist derart dimensioniert, dass bei hoher Belastung des Prothesenmoduls P oder des Prothesenschafts 1 ein Sollbruch an der Sollbruchstelle S erfolgt und nicht an Bauteilen des ersten Kupplungsabschnittes I.

Die Fig. 7c (Schnitt B-B durch das Verbindungsbauteil) und 7d (Schnitt C-C durch das Verbindungsbauteil) zeigen eine jeweils abnehmende Abmessung beziehungsweise Dimensionierung der Sollbruchstelle S. Die Abmessungen der Sollbruchstellen S von Fig. 7b bis 7d nehmen stetig ab, weshalb die nötige Last zur Trennung des Scheibenelements 18 vom Sechskantelement 20 und die damit einhergehende Entkopplung des Prothesenmoduls P vom Prothesenschaft 1 beziehungsweise Entkopplung des ersten vom zweiten Kupplungsabschnittes I, II der Prothesenkupplung 10 stetig abnimmt.

Die Figuren 7b bis 7d zeigen, dass beispielhaft die Dimensionierung der Sollbruchstelle S im Übergangsbereich zwischen Scheibenelement 18 und Sechskantelement 20 durch den Durchmesser D und die Grösse und der Anfasung des zentrisch vorgesehenen Loches L variierbar ist.

An der Sollbruchstelle S kann zusätzlich eine Einkerbung oder Einritzung vorgesehen sein, zur zusätzlichen Schwächung des Verbindungsbauteils V an dieser Stelle.

Durch die oben beschriebenen Gestaltungsmassnahmen ist die Sollbruchstelle S variierbar bzw. wird definiert, bei welcher Krafteinwirkung das Verbindungsbauteil V bricht, womit eine Entkupplung des zweiten Kupplungsabschnittes II vom ersten Kupplungsabschnitt I erfolgt.

Um ein gesichertes Halten des Verbindungsbauteils V im Aufnahmebereich A_{b} zwischen den Verschlusselementen 15', 15" und Innenwänden des Verschlusssockels 14 zu erreichen, müssen die Konturen der Verschlusselemente 15', 15" und des korrespondierenden Verbindungsbauteils V geeignet geformt sein. Beispielsweise können die Verschlusselemente 15', 15" und das Verbindungsbauteil V nur einseitig angefast sein. Der erreichte Hinterschnitt ist ausreichend, um eine Linearbewegung des Verbindungsbauteils V relativ zu den provisorisch gehaltenen Verschlusselementen 15', 15" zu schaffen. Soll das Verbindungsbauteil V und beispielsweise ein daran befestigtes Prothesenmodul P vom ersten Kupplungsabschnitt I abgekuppelt werden, muss ein linearer Zug mit ausreichend hoher Zugkraft weg von der Arretierringseite erfolgen. Durch die Rückstellmittel 17 wird das Verbindungsbauteil V solange im Aufnahmebereich A_{b} gehalten, bis bei ausreichend hoher Zugkraft eine Öffnung der Verschlusselemente 15', 15" aufgrund des Nachgebens der Rückstellmittel 17 erreicht wird. Dann kann das Verbindungsbauteil V von den Verschlusselementen 15', 15" entfernt werden.
Um die mögliche Belastbarkeit der verschiedenen Verbindungsbauteil V einfach zu erkennen, können die Verbindungsbauteile V nach einer Farbkodierung eingefärbt sein. Damit kann die Prothese je nach zu erwartender Belastung mit dem geeigneten Verbindungsbauteil V bzw. der geeigneten Sollbruchstelle S gewählt werden.

Die Einzelteile der Prothesenkupplung 10 können mittels 3D-Drucker aus eher leichtem Material wie Thermoplasten hergestellt werden, beispielsweise aus PLA (Polylaktide), ABS (Acrylnitril-Butadien-Styrol) oder dergleichen. Da die Bauteile relativ zu den aus dem Stand der Technik bekannten Prothesenkupplungen sehr leicht sein können, liegt eine Zielgruppe im Bereich minderjähriger Prothesenträger. Aufgrund des Wachstums sind dort die Zeitabstände für angepasste Bauteile entsprechend kürzer. Aber auch erwachsene Prothesenträger profitieren von der einfachen technischen Gestaltung und dem geringen Gewicht der Prothesenkupplung 10 und einer Prothese mit einer solchen Prothesenkupplung 10.

### Bezugszeichenliste

1 Prothesenschaft
10 Prothesenkupplung
11 Befestigungsring
12 Druckfederelement
13 Arretierring
   131 Führungskugelaufnahme
   132 Führungskugel
   133 Gewindestift
14 Verschlusssockel
   141 Erste Halterille (für das Gummiband 17)
   142', 142" Führungsnut
   143 Führungskanal
144 Erste Führungsstiftaufnahme (am Verschlusssockel 14)
15',15" Verschlusselemente
151', 151" Zweite / Dritte Halterille
152', 152" Führungssteg
153', 153" Zweite / Dritte Führungsstiftaufnahme
16',16" Führungsstifte
17 Rückstellmittel / Gummiband
18 Scheibenelement
19 Rasterscheibenelement
20 Sechskantelement
21 Fixierschrauben (zwischen Verbindungsbauteil und Prothesenmodul)
22 Fixierschrauben (zwischen Prothesenkupplung und Prothesenschaft)
A_{A} Aussparung (Arretierring)
A_{b} Aufnahmebereich (zwischen den Verschlusselementen 15',15")
B Bohrung (Prothesenschaftwand)
E Extremitätenstumpf
F Fase (Verschlusselemente 15',15")
L Loch (im Verbindungsbauteil)
P Prothesenmodul
S Sollbruchstelle
V Verbindungsbauteil
Z Zentriernut
I erster Kupplungsabschnitt
II zweiter Kupplungsabschnitt

## Patentansprüche

1. Prothesenkupplung (10) einer Prothese zur Ankupplung und Abkupplung eines Prothesenmoduls (P) an einem zur Aufnahme eines Extremitätenstumpfs (E) ausgebildeten Prothesenschaft (1), wobei die Prothesenkupplung (10) einen ersten Kupplungsabschnitt (I) und einen zweiten Kupplungsabschnitt (II), welche aneinander an- und abkuppelbar ausgestaltet sind, umfasst, wobei der erste Kupplungsabschnitt (I) einen Verschlusssockel (14) und Verschlusselemente (15', 15") aufweist und ein Aufnahmebereich (A_{b}) am Verschlusssockel (14), von Innenwänden und den Verschlusselementen (15', 15") gebildet ausbildbar ist,
in welchem der zweite Kupplungsabschnitt (II) mit einem Verbindungsbauteil (V) einbringbar ist, wobei die Prothesenkupplung (10) eine Längsachse (X) aufweist,
und wobei das Verbindungsbauteil (V) durch Rückstellkräfte senkrecht zur Längsachse (X) einklemmbar lagerbar ist
und der erste Kupplungsabschnitt (I) einen Arretierring (13) umfasst, welcher linear und/oder rotativ relativ zum Verschlusssockel (14) bewegbar bzw. drehbar in eine Schliessstellung bringbar ist, dass das Verbindungsbauteil (V) arretiert im Aufnahmebereich (A_{b}) lagerbar ist,
**dadurch gekennzeichnet, dass**
die Verschlusselemente (15', 15") bewegbar an Führungsmitteln (142, 144, 16) geführt gehalten sind
und
die Rückstellkräfte durch ein, die Verschlusselemente (15', 15") und den Verschlusssockel (14) mindestens teilweise umgebendes, Rückstellmittel (17) gebildet werden, wodurch im Aufnahmebereich (Ab) eine provisorische Halterung gebildet wird.

2. Prothesenkupplung (10) nach Anspruch 1, wobei Verschlusssockelaussparungen im Verschlusssockel (14) angeordnet sind, sodass die Verschlusselemente (15', 15") derart aufnehmbar sind, dass der äussere Umfang der Verschlusselemente (15', 15") bündig mit dem äusseren Umfang des Verschlusssockels (14) ausbildbar ist.

3. Prothesenkupplung (10) nach einem der vorhergehenden Ansprüche, wobei das Rückstellmittel (17) ein Gummiband (17) ist, welches die Verschlusselemente (15', 15") und den Verschlusssockel (14) umgibt.

4. Prothesenkupplung (10) nach einem der vorhergehenden Ansprüche, wobei im Verschlusssockel (14) mindestens eine Halterille (141) und in den Verschlusselementen (15, 15') zweite und dritte Halterillen (151', 151") ausgespart sind, in welchen das Rückstellmittel (17) gelagert verläuft.

5. Prothesenkupplung (10) nach einem der vorhergehenden Ansprüche, wobei die Verschlusselemente (15, 15') mit Führungsstiftaufnahmen (153', 153") ausgestattet und mit darin angeordneten Führungsstiften (16', 16") als Führungsmittel in einer Richtung senkrecht zu Längsachse (X) bewegbar geführt gelagert sind.

6. Prothesenkupplung (10) nach einem der vorhergehenden Ansprüche, wobei auf der äusseren Umfangsfläche des Verschlusssockels (14) mindestens ein Führungskanal (143) angeordnet ist, in welchem mindestens eine in einer Führungskugelaufnahme (131) im Arretierring (13) gehaltene Führungskugel (132) rollbar gelagert ist, womit der Arretierring (13) von einer Offenstellung in eine Schliessstellung bringbar ist.

7. Prothesenkupplung (10) nach einem der vorhergehenden Ansprüche, wobei der erste Kupplungsabschnitt (I) ein Druckfederelement (12) den Verschlusssockel (14) umschliessend aufweist, welches eine Druckkraft in Richtung der Längsachse (X) auf den Arretierring (13) ausübt.

8. Prothesenkupplung (10) nach einem der vorhergehenden Ansprüche, wobei der Arretierring (13) Aussparungen (A_{A}) entlang seiner Umfangsfläche aufweist, durch welche bei rotativ eingestellter Offenstellung des Arretierringes (13) die Verschlusselemente (15', 15") mindestens teilweise führbar sind.

9. Prothesenkupplung (10) nach einem der vorhergehenden Ansprüche, wobei die Konturen des Verbindungsbauteils (V) und die dem Verbindungsbauteil (V) zugewandten Konturen der Verschlusselemente (15', 15") Fasen (F) zur Erzeugung eines Hinterschnitts aufweisen.

10. Prothesenkupplung (10) nach Anspruch 9, wobei die Konturen des Verbindungsbauteils (V) und der Verschlusselemente (15', 15") eine Zentriernut (Z) aufweisen.

11. Prothesenkupplung (10) nach einem der vorhergehenden Ansprüche, wobei das Verbindungsbauteil (V) ein Mehrkantelement (20) mit einer Querschnittsfläche als Polygon mit mindestens drei Eckpunkten ausgeführt ist.

12. Prothesenkupplung (10) nach Anspruch 11, wobei ein Scheibenelement (18) am Mehrkantelement (20) angeformt ist und im Übergangsbereich zwischen dem Mehrkantelement (20) und dem Scheibenelement (18) eine Sollbruchstelle (S) ausgebildet ist.

13. Prothesenkupplung (10) nach Anspruch 12, wobei das Scheibenelement (18) vollständig querend konzentrisch beabstandet vom Mehrkantelement (20) angeordnete Befestigungsaussparungen aufweist.

14. Prothese mit einer Prothesenkupplung (10) nach einem der vorhergehenden Ansprüche, wobei das Verbindungsbauteil (V) des zweiten Kupplungsabschnitts (II) an einem Prothesenmodul (P) und der erste Kupplungsabschnitt (I) mit einem Befestigungsring (11) an einem Prothesenschaft (1) fixiert ist, sodass eine Ankupplung und Abkupplung des Prothesenmoduls (P) am Prothesenschaft (1) erzielbar ist.

15. Prothese nach Anspruch 14, wobei das Verbindungsbauteil (V) ein Mehrkantelement (20) mit einer Querschnittsfläche als Polygon mit mindestens drei Eckpunkten ausgeführt ist, an welchem ein Scheibenelement (18) angeformt ist und im Übergangsbereich zwischen dem Mehrkantelement (20) und dem Scheibenelement (18) eine Sollbruchstelle (S) ausgebildet ist.

## Claims

1. Prosthesis coupler (10) of a prosthesis for coupling and uncoupling a prosthesis module (P) on a prosthesis shaft (1) formed to receive an extremity stump (E), wherein the prosthesis coupler (10) comprises a first coupling section (I) and a second coupling section (II), which are designed to be coupled to and uncoupled from each other, wherein the first coupling section (I) has a locking socket (14) and locking elements (15', 15") and a receiving area (A_{b}), formed of inner walls and the locking elements (15', 15"), can be produced on the locking socket (14), into which the second coupling section (II) with a connection component (V) can be brought, wherein the prosthesis coupler (10) has a longitudinal axis (X) and wherein the connection component (V) can be supported in a clamped manner by restoring forces perpendicular to the longitudinal axis (X)
and the first coupling section (I) comprises a locking ring (13) which in a linear and/or rotative manner relative to the locking socket (14) can be moved or turned into a locking position, and the connection component (V) can be borne locked in the receiving area (A_{b})
**characterised in that**
the locking elements (15', 15") are held movably in a guided manner on guiding means (142, 144, 16)
and
the restoring forces are produced by a restoring means (17) at least partially surrounding the locking elements (15', 15") and the locking socket (14), through which a provisional fixture is formed in the receiving area (A_{b}).

2. Prosthesis coupler (10) according to claim 1, wherein locking socket recesses are arranged in the locking socket (14) so that the locking elements (15', 15") can be held in such a way that the outer perimeter of the locking elements (15', 15'') can be configured to be flush with the outer perimeter of the locking socket (14).

3. Prosthesis coupler (10) according to any one of the preceding claims, wherein the restoring means (17) is a rubber band (17) which surrounds the locking elements (15', 15") and the locking socket (14).

4. Prosthesis coupler (10) according to any one of the preceding claims, wherein in the locking socket (14) at least one retaining grove (141) and in the locking elements (15, 15') second and third retaining grooves (151', 151") are recessed, in which the restoring means (17) is borne.

5. Prosthesis coupler (10) according to any one of the preceding claims, wherein the locking elements (15, 15') are provided with guide pin holders (153', 153'') and, with guide pins (16', 16") arranged therein as guiding means, are movably borne in a direction perpendicular to the longitudinal axis (X).

6. Prosthesis coupler (10) according to any one of the preceding claims, wherein on the outer perimeter surface of the locking socket (14) at least one guide channel (143) is arranged in which at least one guide ball (132), held in a guide ball holder (131) in the locking ring (13), is borne in a rolling manner, with which the locking ring (13) can be brought from an open position into a locking position.

7. Prosthesis coupler (10) according to any one of the preceding claims, wherein the first coupling section (I) comprises a compression spring element (12) which surrounds the locking socket (14) and exerts a pressure force on the locking ring (13) in the direction of the longitudinal axis (X).

8. Prosthesis coupler (10) according to any one of the preceding claims, wherein the locking ring (13) has recesses (A_{A}) along its perimeter surface, through which in the rotatively set open position of the locking ring (13) the locking elements (15', 15") are at least partially guidable.

9. Prosthesis coupler (10) according to any one of the preceding claims wherein the contours of the connection component (V) and the contours of the locking elements (15', 15") facing the connection component (V) comprise chamfers (F) to form an undercut.

10. Prosthesis coupler (10) according to claim 9 wherein the contours of the connection component (V) and the locking elements (15', 15") have a centring groove (Z) .

11. Prosthesis coupler (10) according to any one of the preceding claims, wherein the connection component (V) is configured as a polygonal element (20) with a cross-sectional area as a polygon with at least corner points.

12. Prosthesis coupler (10) according to claim 11 wherein a disk element (18) is formed on the polygonal element (20) and in the transition area between the polygonal element (20) and the disk element (18) there is a predetermined breaking point (S).

13. Prosthesis coupler (10) according to claim 12, wherein the disk element (18) comprises completely traversing fastening recesses arranged concentrically at a distance from the polygonal element (20).

14. Prosthesis with a prosthesis coupler (10) according to any one of the preceding claims, wherein the connection component (V) of the second coupling section (II) is fixed to a prosthesis module (P) and the first coupling section (I) is fixed with a fastening ring (11) to a prosthesis shaft (1), so that coupling and uncoupling of the prosthesis module (P) to and from the prosthesis shaft (1) can be achieved.

15. Prosthesis according to claim 14, wherein the connection component (V) is configured as a polygonal element (20) with a cross-sectional area as a polygon with at least three corner points, on which a disk element (18) is formed, and in the transition area between the polygonal element (20) and the disc element (18) a predetermined break point (S) is provided.

## Revendications

1. Dispositif d'accouplement de prothèse (10) conçu pour une prothèse, destiné à accoupler et à désaccoupler un module prothétique (P) sur une tige prothétique (1), configurée pour recevoir un moignon d'extrémité (E), le dispositif d'accouplement de prothèse (10) comprenant une première partie d'accouplement (I) et une deuxième partie d'accouplement (II), conçues pour être accouplées l'une à l'autre et désaccouplées, la première partie d'accouplement (I) comportant une base de verrouillage (14) et des éléments de verrouillage (15', 15") et une zone de réception (A_{b}) sur la base de verrouillage (14), dans laquelle la deuxième partie d'accouplement (II) peut être introduite avec une pièce de liaison (V) pouvant être conçue en étant formée par des parois internes et par les éléments de verrouillage (15', 15"), le dispositif d'accouplement de prothèse (10) comportant un axe longitudinal (X) et la pièce de liaison (V) étant susceptible d'être logée en étant serrée par des forces de rappel à la perpendiculaire de l'axe longitudinal (X)
et la première partie d'accouplement (I) comprenant une bague de blocage (13), laquelle en étant mobile ou rotative de manière linéaire et/ou en rotation par rapport à la base de verrouillage (14) est susceptible d'être amenée dans une position de fermeture, en ce que la pièce de liaison (V) est susceptible d'être logée en étant bloquée dans la zone de réception (A_{b}),
**caractérisé en ce que**
les éléments de verrouillage (15', 15") sont maintenus en étant guidés de manière mobile sur des moyens de guidage (142, 144, 16)
et
les forces de rappel sont créées par un moyen de rappel (17) entourant au moins partiellement les éléments de verrouillage (15', 15") et la base de verrouillage (14), suite à quoi, une attache provisoire est créée dans la zone de réception (A_{b}).

2. Dispositif d'accouplement de prothèse (10) selon la revendication 1, des encoches de base de verrouillage étant placées dans la base de verrouillage (14), de telle sorte que les éléments de verrouillage (15', 15") puissent être réceptionnés de manière à ce que la périphérie extérieure des éléments de verrouillage (15', 15") puisse être conçue à fleur de la périphérie extérieure de la base de verrouillage (14).

3. Dispositif d'accouplement de prothèse (10) selon l'une quelconque des revendications précédentes, le moyen de rappel (17) étant une bande de caoutchouc (17), laquelle entoure les éléments de verrouillage (15', 15") et la base de verrouillage (14).

4. Dispositif d'accouplement de prothèse (10) selon l'une quelconque des revendications précédentes, dans la base de verrouillage (14) étant ménagée au moins une rainure de retenue (141) et dans les éléments de verrouillage (15, 15') étant ménagées des deuxièmes et troisièmes rainures des retenue (151', 151"), dans lesquelles le moyen de rappel (17) est logé en s'écoulant.

5. Dispositif d'accouplement de prothèse (10) selon l'une quelconque des revendications précédentes, les éléments de verrouillage (15, 15') étant équipés de logements de goupilles de guidage (153', 153") et par l'intermédiaire de goujons de guidage (16', 16") placés dans ces derniers en tant que moyens de guidage, étant logés en étant guidés de manière mobile dans une direction à la perpendiculaire de l'axe longitudinal (X).

6. Dispositif d'accouplement de prothèse (10) selon l'une quelconque des revendications précédentes, sur la surface périphérique extérieure de la base de verrouillage (14) étant placé au moins un canal de guidage (143) dans lequel est logée en roulement au moins une bille de guidage (132) maintenue dans un logement de bille de guidage (131) dans la bague de blocage (13), à l'aide de laquelle la bague de blocage (13) peut être amenée d'une position d'ouverture dans une position de fermeture.

7. Dispositif d'accouplement de prothèse (10) selon l'une quelconque des revendications précédentes, la première partie d'accouplement (I) comportant un élément à ressort de pression (12) entourant la base de verrouillage (14), lequel exerce une force de pression sur la bague de blocage (13), dans la direction de l'axe longitudinal (X).

8. Dispositif d'accouplement de prothèse (10) selon l'une quelconque des revendications précédentes, la bague de blocage (13) comportant des encoches (A_{A}) le long de sa surface périphérique, à travers lesquelles, dans une position d'ouverture réglée de manière rotative de la bague d'arrêt (13), les éléments de verrouillage (15', 15") peuvent être guidés au moins partiellement.

9. Dispositif d'accouplement de prothèse (10) selon l'une quelconque des revendications précédentes, les contours de la pièce de liaison (V) et les contours des éléments de verrouillage (15', 15") qui font face à la pièce de liaison (V) comportant des chanfreins (F), destinés à créer une contre-dépouille.

10. Dispositif d'accouplement de prothèse (10) selon la revendication 9, les contours de la pièce de liaison (V) et des éléments de verrouillage (15', 15") comportant une rainure de centrage (Z).

11. Dispositif d'accouplement de prothèse (10) selon l'une quelconque des revendications précédentes, la pièce de liaison (V) étant réalisée sous la forme d'un élément polygonal (20) pourvu d'une surface de section transversale en polygone présentant au moins trois points d'angle.

12. Dispositif d'accouplement de prothèse (10) selon la revendication 11, un élément en forme de disque (18) étant surmoulé sur l'élément polygonal (20) et dans la zone de passage entre l'élément polygonal (20) et l'élément en forme de disque (18) étant conçue une zone de rupture théorique (S).

13. Dispositif d'accouplement de prothèse (10) selon la revendication 12, l'élément en forme de disque (18) comportant des encoches de fixation écartées de manière concentrique sur toute l'étendue transversale de l'élément polygonal (20).

14. Prothèse, dotée d'un dispositif d'accouplement de prothèse (10) selon l'une quelconque des revendications précédentes, la pièce de liaison (V) de la deuxième partie d'accouplement (II) étant fixée sur un module prothétique (P) et la première partie d'accouplement (I) étant fixée à l'aide d'une bague de fixation (11) sur une tige prothétique (1), de sorte à pouvoir obtenir un accouplement et un désaccouplement du module prothétique (P) sur la tige prothétique (1).

15. Prothèse selon la revendication 14, la pièce de liaison (V) étant réalisée sous la forme d'un élément polygonal (20) avec une surface de section transversale en forme de polygone, présentant au moins trois points d'angle, sur lequel est surmoulé un élément en forme de disque (18) et dans la zone de passage entre l'élément polygonal (20) et l'élément en forme de disque (18) étant conçue une zone de rupture théorique (S).
